# EUROPÄISCHE PATENTSCHRIFT

(11) **EP 3 285 836 B1**
(45) Veröffentlichungstag und Bekanntmachung des Hinweises auf die Patenterteilung: **27.03.2019**
(21) Anmeldenummer: 16720381.9
(22) Anmeldetag: 21.04.2016
(51) Int. Cl.: A61M 5/31, C23C 16/40, A61M 5/19, A61F 9/00

(54) **PRIMÄRPACKMITTEL UND VERFAHREN ZUR HERSTELLUNG EINES PRIMÄRPACKMITTELS**
PRIMARY PACKAGING MATERIAL AND METHOD FOR THE PRODUCTION OF A PRIMARY PACKAGING MATERIAL
MOYEN D'EMBALLAGE PRINCIPAL ET PROCÉDÉ DE FABRICATION D'UN MOYEN D'EMBALLAGE PRINCIPAL

(30) Priorität: 21.04.2015 DE 102015207228
(43) Veröffentlichungstag der Anmeldung: 28.02.2018
(73) Patentinhaber: Vetter Pharma-Fertigung GmbH & Co. KG, 88212 Ravensburg (DE)
(72) Erfinder: KÜHNLE, Sarah, 88045 Friedrichshafen (DE); BÖTTGER, Frank, 88214 Ravensburg (DE); NELLES, Paul, 88213 Ravensburg (DE)
(74) Vertreter: Kordel, Mattias
(86) Internationale Anmeldenummer: PCT/EP2016/058895
(87) Internationale Veröffentlichungsnummer: WO 2016/170054

(56) Entgegenhaltungen:
- EP-A2- 0 920 879
- WO-A1-2014/065359
- US-A1- 2005 192 530
- US-A1- 2013 041 241

## Beschreibung

Die Erfindung betrifft ein Primärpackmittel für medizinische Zubereitungen nach Anspruch 1 sowie ein Verfahren zur Herstellung eines solchen Primärpackmittels nach Anspruch 9.

Primärpackmittel, insbesondere vorgefüllte Spritzen- oder Doppelkammersysteme, werden zur Lagerung und zur parenteralen Administration von Wirk- und/oder Hilfsstoffen verwendet. Ein aus dem Stand der Technik bekanntes Primärpackmittel ist in US 2013/041241 A1 offenbart. Dabei existieren Anwendungen, welche neben einer ohnehin erforderlichen Innensterilität des Primärpackmittels auch eine Außensterilität, also insbesondere eine Sterilität der äußeren Oberfläche des Primärpackmittels, erforderlich machen. Dies betrifft insbesondere Primärpackmittel für ophthalmische Darreichungen zu Injektionszwecken, welche in jüngster Zeit durch die Zulassung neuer, moderner Medikamente zur Behandlung der makularen Degeneration eine erhöhte Bedeutung erlangen. Solche Primärpackmittel müssen in einer Sekundärverpackung, beispielsweise einem Blister, einer Behandlung unterzogen werden, welche die Außensterilität gewährleistet. Dabei scheiden thermische oder strahlungsintensive Verfahren aus, da ansonsten der Wirkstoff in dem Primärpackmittel beschädigt werden würde. Aus diesem Grund werden typischerweise chemische Sterilisationen, wie beispielsweise eine Behandlung mit Ethylenoxid oder mit vaporisiertem Wasserstoffperoxid, gewählt. Das Primärpackmittel muss dabei gegenüber einem Eindringen solcher Reagenzien ausreichend dicht sein, um eine Kontamination des Inhalts zu vermeiden. Dies erschwert generell die Wahl der Materialien für das Primärpackmittel. Primärpackmittel aus Glas genügen den genannten Anforderungen, bedingen jedoch einen sehr hohen Entwicklungs- und Kontrollaufwand, insbesondere um eine noch tolerierbare Anzahl von subvisuellen Partikeln in einer zu injizierenden Lösung zu erreichen. Dies ist ganz besonders dann der Fall, wenn solche Glaskörper in ihrem Inneren zur Verbesserung der Gleiteigenschaften eines Stopfens silikonisiert sind. Hinzu tritt die Gefahr, welche von sich ablösenden, mikroskopischen Glasteilchen ausgeht, die bei einer Lagerung oder beim Transport entstehen, jedoch mit bloßem Auge nicht erkannt werden können. Die hier genannten, spezifischen Nachteile von Glas-Primärpackmittel können durch Primärpackmittel aus Kunststoff zwar umgangen werden, dafür haben typischerweise verwendete, hochtransparente Kunststoffe den Nachteil, gasdurchlässig zu sein.

Der Erfindung liegt die Aufgabe zugrunde, ein Primärpackmittel sowie ein Verfahren zur Herstellung eines solchen Primärpackmittels zu schaffen, wobei die genannten Nachteile nicht auftreten.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem ein Primärpackmittel für medizinische Zubereitung geschaffen wird, das einen Grundkörper aufweist, der einen Kunststoff aufweist oder aus einem Kunststoff gebildet ist, wobei der Grundkörper wenigstens eine Wandung mit einer Außenfläche und wenigstens einer Innenfläche aufweist. Dabei ist die Außenfläche einem Äußeren des Primärpackmittels zugewandt, wobei die Innenfläche einem Innenraum des Primärpackmittels, insbesondere einem Innenraum zur Aufnahme eines Wirk- und/oder Hilfsstoffs oder eines Lösungsmittels, zugewandt ist. Es ist insbesondere möglich, dass das Primärpackmittel eine zylindrische Wandung aufweist, welche eine äußere Mantelfläche und eine innere Umfangsfläche aufweist. Das Primärpackmittel zeichnet sich dadurch aus, dass die Außenfläche mit einer gasdichten Beschichtung versehen ist. Das Primärpackmittel weist Vorteile gegenüber dem Stand der Technik auf. Insbesondere verwirklichen sich die Vorteile, welche generell mit einem aus Kunststoff gebildeten Grundkörper im Vergleich zu einem Glas-Primärpackmittel verbunden sind, wobei nämlich eine deutlich geringere Anzahl von subvisuellen Partikeln in Lösungen - wenn überhaupt solche auftreten - zu befürchten ist, sodass auch der mit dem Primärpackmittel verbundene Entwicklungs- und Kontrollaufwand deutlich geringer ist. Weiterhin besteht nicht die Gefahr von sich ablösenden, mikroskopischen Glasteilchen. Die an der Außenfläche vorgesehene, gasdichte Beschichtung verleiht dem Primärpackmittel impermeable Eigenschaften, insbesondere gegenüber Reagenzien, die für eine chemische Sterilisation verwendet werden. Daher kann für das Primärpackmittel eine Außensterilität durch chemische Sterilisation, beispielsweise mit Ethylenoxid oder vaporisiertem Wasserstoffperoxid, sichergestellt werden. Dabei ist das Primärpackmittel gegenüber einem Eindringen dieser Reagenzien aufgrund der gasdichten Beschichtung der Außenfläche ausreichend dicht.

Vorzugsweise ist die gesamte Außenfläche des Grundkörpers - insbesondere nahtlos - mit der gasdichten Beschichtung versehen. Dadurch ist es ausgeschlossen, dass bei einer Terminalsterilisation Sterilisationsmittelrückstande über etwaige ungeschützte Kunststoffbereiche in das Innere des Primärpackmittels diffundieren können.

Vorzugsweise ist die gasdichte Beschichtung an der Außenfläche als immobilisierte Schicht vorgesehen. Diese ist somit langzeitstabil und unveränderlich, sowie fest und sicher an der Außenfläche angeordnet.

Die gasdichte Beschichtung weist besonders bevorzugt impermeable Eigenschaften gegenüber Sauerstoff, Kohlendioxid, Ethylenoxid, und/oder Wasserstoffperoxid auf. Hierbei handelt es sich um besonders gängige Reagenzien, die für eine chemische Terminalsterilisation zur Gewährleistung der Außensterilität des Primärpackmittels eingesetzt werden.

Es wird ein Ausführungsbeispiele des Primärpackmittels bevorzugt, das sich dadurch auszeichnet, dass die gasdichte Beschichtung eine Schicht aufweist, die ein Metall oder ein Halbmetall aufweist, oder aus einem Metall oder einem Halbmetall besteht. Insbesondere weist die Schicht bevorzugt einen Metall- oder Halbmetallfilm auf oder besteht aus einem Metall- oder Halbmetallfilm. Dabei kann die Schicht insbesondere Gold, Aluminium, Chrom, Silber oder ein anderes geeignetes Metall oder Halbmetall aufweisen, oder aus einem der genannten Metalle oder einem anderen geeigneten Metall oder Halbmetall bestehen.

Alternativ oder zusätzlich ist es möglich, dass die gasdichte Beschichtung eine Schicht aufweist, die Silizium oder eine Siliziumverbindung - vorzugsweise mit quarz- oder glasartiger Zusammensetzung - aufweist, oder aus Silizium oder einer Siliziumverbindung - vorzugsweise mit quarz- oder glasartiger Zusammensetzung - besteht. Besonders bevorzugt weist die Schicht einen aus einer Siliziumverbindung aufgebauten Film mit glasartiger Zusammensetzung auf oder besteht aus einem solchen Film. Dazu zählen insbesondere quarzartige Beschichtungen.

Eine solche Schicht aus Metall oder einem Halbmetall, oder aber aus Silizium oder einer Siliziumverbindung - insbesondere mit glasartiger Zusammensetzung - stellt die gewünschten Gasbarriereeigenschaften bereit. Die gasdichte Beschichtung kann dabei insbesondere eine Mehrzahl gleichartiger oder verschiedener, insbesondere in ihrer Zusammensetzung geringfügig verschiedener Teilschichten aufweisen oder aus solchen Teilschichten bestehen.

Es ist auch möglich, dass die gasdichte Beschichtung Diamant aufweist oder aus Diamant besteht. Alternativ oder zusätzlich ist es möglich, dass die gasdichte Beschichtung Teflon aufweist oder aus Teflon besteht.

Es wird auch ein Ausführungsbeispiel des Primärpackmittels bevorzugt, dass sich dadurch auszeichnet, dass der Grundkörper einen durchsichtigen Kunststoff aufweist oder aus einem durchsichtigen Kunststoff besteht. Der durchsichtige Kunststoff ist vorzugsweise ausgewählt aus einer Gruppe bestehend aus einem zyklischen Olefin-Polymer (COP), einem zyklischen Olefin-Copolymer (COC) und Polycarbonat (PC). Diese Materialien eignen sich besonders zur Herstellung insbesondere transparenter Grundkörper für ein Primärpackmittel.

Es wird auch ein Ausführungsbeispiel des Primärpackmittels bevorzugt, das sich dadurch auszeichnet, dass zwischen der Außenfläche und der gasdichten Beschichtung eine Haftvermittlungsschicht angeordnet ist. Dabei dient die Haftvermittlungsschicht einer verbesserten Haftung der gasdichten Beschichtung auf der Außenfläche. Insbesondere ermöglicht die Haftvermittlungsschicht vorzugsweise eine kovalente Bindung der auf ihr aufbauenden gasdichten Beschichtung.

Es wird auch ein Ausführungsbeispiel des Primärpackmittels bevorzugt, das sich dadurch auszeichnet, dass auf der gasdichten Beschichtung zumindest bereichsweise oder auch insgesamt eine Funktionsschicht angeordnet ist. Die Funktionsschicht dient dabei der Bereitstellung einer spezifischen Funktion oder einer - insbesondere lokalen - Verbesserung der Eigenschaften einer äußeren Fläche der gasdichten Beschichtung für eine bestimmte Funktion. Dabei kann die Funktionsschicht beispielsweise vorgesehen sein, um eine bessere Haftung für Klebe-Etiketten bereitzustellen. Insbesondere kann es sich dabei um eine immobilisierte Schicht handeln, welche Eigenschaften aufweist, die unterbinden, dass Inhaltstoffe von Außenmarkierungen oder Etiketten, insbesondere Klebe-Etiketten, in das abgefüllte Produkt migrieren können. Die Funktionsschicht kann auch vorgesehen sein, um Farbstoffe oder Markierungen aufzubringen, wobei sie hierzu insbesondere bessere Eigenschaften aufweist, als die gasdichte Beschichtung selbst. Zusätzlich oder alternativ kann eine Funktionsschicht vorgesehen sein, welche eine im Vergleich zu der gasdichten Beschichtung verringerte Empfindlichkeit gegenüber Kratzern oder kosmetischen Defekten aufweist.

Es ist möglich, dass das Primärpackmittel eine Mehrzahl von Funktionsschichten aufweist, die zu verschiedenen Zwecken eingerichtet und vorgesehen sein können, wobei insbesondere lokal an verschiedenen Stellen des Primärpackmittels verschiedene Funktionsschichten vorgesehen sein können. Es ist auch möglich, dass verschiedene Funktionsschichten übereinander angeordnet sind, wobei sie so kumulativ an einer Stelle des Primärpackmittels verschiedene Funktionen gewährleisten können.

Die wenigstens eine Funktionsschicht ist vorzugsweise als immobilisierte Schicht ausgebildet und so besonders stabil, fest und sicher auf der gasdichten Beschichtung angeordnet. Es ist möglich, dass sowohl die gasdichte Beschichtung als auch die wenigstens eine Funktionsschicht Silizium oder eine Siliziumverbindung - insbesondere mit quarz- oder glasartiger Zusammensetzung - aufweisen. Dabei ist es möglich, dass sich die gasdichte Beschichtung und die wenigstens eine Funktionsschicht in einem Sauerstoff-Gehalt unterscheiden. Insbesondere kann es sich bei beiden Schichten um quarzartige Schichten, allerdings mit verschiedenem Sauerstoff-Gehalt, handeln.

Es ist insbesondere möglich, dass der Sauerstoff-Gehalt der wenigstens einen Funktionsschicht höher ist als der Sauerstoff-Gehalt der gasdichten Beschichtung. Je höher der Sauerstoff-Gehalt der quarzartigen Beschichtung ist, desto glasartiger ist sie. Dabei zeigt sich, dass beispielsweise Klebe-Etiketten optimiert sind für eine Klebeverbindung mit Glas. Daher ist es günstig, jedenfalls in einem Bereich, in dem ein Klebe-Etikett aufgeklebt werden soll, auf der gasdichten Beschichtung eine möglichst glasartige Funktionsschicht vorzusehen, beispielsweise indem dort - insbesondere lokal - der Sauerstoff-Gehalt der Beschichtung erhöht ist.

Entsprechend der Erfindung zeichnet sich das Primärpackmittel dadurch aus, dass die Innenfläche mit einer gasdichten Innenbeschichtung versehen ist. Hierdurch kann die Gasdichtheit des Primärpackmittels zusätzlich erhöht werden, weil die gasdichte Beschichtung der Außenfläche und die gasdichte Innenbeschichtung gemeinsam eine besonders hohe Dichtigkeit gegenüber gasförmigen Stoffen, insbesondere solchen zur chemischen Sterilisation, bereitstellen. Vorzugsweise ist die gasdichte Innenbeschichtung als immobilisierte Schicht vorgesehen.

Besonders bevorzugt weist die gasdichte Innenbeschichtung wenigstens eine der Eigenschaften auf, welche zuvor für die gasdichte Beschichtung der Außenfläche beschrieben wurden. Insbesondere weist sie bevorzugt wenigstens eine Schicht auf, die ein Metall oder Halbmetall aufweist oder aus einem Metall oder Halbmetall besteht, oder die Silizium oder eine Siliziumverbindung - vorzugsweise mit quarz- oder glasartiger Zusammensetzung - aufweist oder aus Silizium oder einer Siliziumverbindung - vorzugsweise mit quarz- oder glasartiger Zusammensetzung - besteht. Weiterhin ist zwischen der Innenfläche und der gasdichten Innenbeschichtung vorzugsweise eine Haftvermittlungsschicht angeordnet.

Für die gasdichte Innenbeschichtung kommen insbesondere Metall- oder Halbmetallfilme, beispielsweise aus Gold, Aluminium, Silber, Chrom oder einem anderen geeigneten Material, und/oder aus Silizium oder einer Siliziumverbindung aufgebaute Filme, insbesondere mit glasartiger oder quarzartiger Zusammensetzung, infrage. Es ist auch möglich, dass die gasdichte Innenbeschichtung aus einer Mehrzahl gleichartiger oder - insbesondere in Hinblick auf deren chemische Zusammensetzung - leicht verschiedenen Teilschichten besteht.

Es ist auch möglich, dass die gasdichte Innenbeschichtung Diamant aufweist oder aus Diamant besteht. Alternativ oder zusätzlich ist es möglich, dass die gasdichte Innenbeschichtung Teflon aufweist oder aus Teflon besteht.

Vorzugsweise ist auch die Innenfläche der Wandung des Grundkörpers insgesamt und insbesondere nahtlos mit der gasdichten Innenbeschichtung versehen. Es ist aber auch möglich, die gasdichte Innenbeschichtung lokal oder bereichsweise auszusparen.

Im Einklang mit der Erfindung zeichnet sich das Primärpackmittel dadurch aus, dass auf der gasdichten Innenbeschichtung zumindest bereichsweise eine Innenfunktionsschicht angeordnet ist. Dabei handelt es sich vorzugsweise um eine immobilisierte Schicht. Es zeigt sich nämlich, dass ein abgefüllter Wirk- und/oder Hilfsstoff während der Abfüllung und während der Lagerung im abgefüllten Zustand frei von partikulären Verunreinigungen sein muss. Insbesondere bei ophthalmischen Darreichungen drohen sonst selbst kleinste Partikel im subvisuellen Bereich, die Sehfähigkeit des Patienten dauerhaft zu beeinträchtigen. Die Innenfunktionsschicht kann auf der gesamten gasdichten Innenbeschichtung, aber auch nur bereichsweise oder lokal vorgesehen sein.

Darüber hinaus besteht die Gefahr, dass ein in Kontakt mit einem Wirk- und/oder Hilfsstoff stehendes Wandungsmaterial des Primärpackmittels bei einer aseptischen Gefriertrocknung verwittert, wodurch wiederum partikuläre Verunreinigungen in den Wirk- und/oder Hilfsstoff gelangen können. Diese Gefahren und/oder Nachteile können insbesondere mit einer immobilisierten Innenfunktionsschicht vermieden werden, die stabil ist gegenüber Verwitterung und Abrieb.

Zusätzlich oder alternativ ist bevorzugt eine Innenfunktionsschicht vorgesehen, welche eine Haftreibung von Elastomerstoffen herabsetzt, wobei sie hierdurch insbesondere eine vereinfachte Dosierung von kleinen Volumina ermöglicht. Außerdem bedarf es dann keiner Silikonisierung der Innenfläche, sodass hierdurch eingetragene partikuläre Verunreinigungen vermieden werden.

Besonders bevorzugt ist die Innenfunktionsschicht so ausgebildet, dass sie keine messbaren Partikel oder Fremdstoffe, wie beispielsweise Silikontropfen, an den Wirk- und/oder Hilfsstoff abgibt.

Besonders bevorzugt ist die wenigstens eine Innenfunktionsschicht als - insbesondere impermeabler - Gleitfilm ausgebildet.

Bevorzugt ist/sind die gasdichte Innenbeschichtung und/oder die Innenfunktionsschicht bereichsweise, insbesondere lokal, ausgespart. Dies bedeutet, dass in wenigstens einem - vorzugsweise ringförmig umlaufenden - Freibereich der Innenfläche keine gasdichte Innenbeschichtung und/oder keine Innenfunktionsschicht angeordnet ist. Der Freibereich ist demnach frei von der gasdichten Innenbeschichtung und/oder der Innenfunktionsschicht. Das Primärpackmittel weist als Freibereich bevorzugt einen Bereich auf, in dem bestimmungsgemäß beim Befüllen und Gefriertrocknen des Primärpackmittels ein Lyophilisat angeordnet wird. Besonders bevorzugt wird eine solche Ausgestaltung für ein als Doppelkammersystem ausgebildetes Primärpackmittel, das vorzugsweise eingerichtet ist für eine ophthalmische Anwendung. Bei einem Doppelkammersystem ist der Freibereich bevorzugt ein ringförmiger Bereich distal oberhalb eines Mittelstopfenbereichs, in dem bestimmungsgemäß ein Mittelstopfen angeordnet wird.

Besonders bevorzugt wird ein Ausführungsbeispiel, bei welchem die Innenfläche der Wandung des Grundkörpers insgesamt und insbesondere nahtlos mit der gasdichten Innenbeschichtung versehen ist. Entsprechend der vorliegenden Erfindung ist die Innenfunktionsschicht in wenigstens einem Freibereich ausgespart.

Ein weitere Möglichkeit besteht nach allem darin, die innenliegende Beschichtung - vornehmlich eine Gleitschicht - in einem Teilbereich auszusparen. Dies ist insbesondere für den Bereich von Vorteil, in welchem eine zu gefriertrocknende Produktlösung in Kontakt mit der Behälterwandung und gegebenenfalls der Beschichtung kommt. Dadurch kann grundsätzlich vermieden werden, dass Interaktionen zwischen Produkt und Beschichtung während des Herstellungsprozesses, insbesondere während der Gefriertrocknung, des finalen Arzneimittels und dessen Lagerung stattfinden, welche die Produktqualität beeinträchtigen. Bei ophthalmischen Produkten unerwünschte Interaktionen könnten beispielsweise Silikonöltropfen in Lösung oder das Auftreten von Beschichtungsfragmenten zur Folge haben. Zusätzlich oder alternativ ist bevorzugt eine Innenfunktionsschicht vorgesehen, die - je nach Bedarf bei einem konkreten Primärpackmittel - hydrophobe oder hydrophile Eigenschaften aufweist. Eine hydrophobe Schicht weist vorzugsweise Polydimethylsiloxan oder eine perfluorierte Verbindung, beispielsweise Polytetrafluorethylen (PTFE), auf oder besteht aus einem der genannten Stoffe. Insbesondere weist die hydrophobe Schicht vorzugsweise einen Film auf, welcher einen der genannten Stoffe aufweist oder daraus besteht. Eine hydrophile Innenfunktionsschicht weist vorzugsweise Carboxylgruppen auf, wobei eine solche hydrophile Innenfunktionsschicht insbesondere durch plasma-induzierte Polymerisation von Acrylsäure hergestellt werden kann.

Generell dienen solche hydrophoben oder hydrophilen Innenfunktionsschichten einer Reduktion von Haftreibung sowie insbesondere der vollständigen Entleerbarkeit, wobei für die vollständige Entleerbarkeit eines wässrigen Wirk- und/oder Hilfsstoffs eine hydrophobe Innenfunktionsschicht vorgesehen sein kann, wobei für die vollständige Entleerbarkeit eines lipophilen Wirk- und/oder Hilfsstoffs eine hydrophile Innenfunktionsschicht vorgesehen sein kann.

Die wenigstens eine Innenfunktionsschicht weist vorzugsweise eine Quervernetzbarkeit oder Quervernetzung auf, insbesondere in Form zusätzlicher funktioneller Gruppen, reaktiver Gruppen, radikalischer Gruppen, ionischer Gruppen, Doppelbindungen, oder anderer funktioneller Gruppen, welche miteinander vernetzt werden können, insbesondere durch UV-Bestrahlung der Innenfunktionsschicht. Hierdurch kann die Molmasse der Innenfunktionsschicht erhöht werden, wodurch diese besonders immobil ausgestaltet werden kann, sodass ein Ablösen oder eine Migration der Innenfunktionsschicht in den Wirk- und/oder Hilfsstoff verhindert werden kann.

Besonders bevorzugt wird ein Ausführungsbeispiel des Primärpackmittels, das sich dadurch auszeichnet, dass dieses als Doppelkammersystem ausgebildet ist. Es kann dabei insbesondere als Doppelkammerkarpule oder als Doppelkammerspritze ausgebildet sein, wobei es eine erste Kammer für eine medizinische Darreichung in lyophilisierter, pulverförmiger oder flüssiger Form aufweist. In einer zweiten Kammer befindet sich eine flüssige Form, insbesondere ein Lösungsmittel, welche kurz vor einer Verabreichung zum Auflösen der festen medizinischen Darreichung in der ersten Kammer oder einer zu einer Injektion zeitnahen Durchmischung mit einer in der ersten Kammer vorliegenden flüssigen medizinischen Darreichung dient.

Alternativ ist es möglich, dass das Primärpackmittel als Einkammerkarpule oder als Einkammerspritze ausgebildet ist.

Besonders bevorzugt wird ein Ausführungsbeispiel des Primärpackmittels, welches eingerichtet ist für eine ophthalmische Anwendung. Dabei zeichnet sich das Primärpackmittel insbesondere durch ein geringen Füllvolumen von in der Regel weniger als oder höchstens 200 µl aus. Dies ist deswegen der Fall, da typischerweise nicht mehr als 50 µl in den Glaskörper des menschlichen Auges injiziert werden sollen. Vorzugsweise sind an dem Primärpackmittel Außenmarkierungen angeordnet, um einem Arzt eine vereinfachte Dosierung zu ermöglichen. Ein geeignet dimensioniertes Format für das Primärpackmittel ist ein Volumen von 1 ml oder 0,5 ml. Ophthalmische Darreichungen zu Injektionszwecken erfahren in jüngster Zeit eine erhöhte Bedeutung durch die Zulassung neuer, moderner Medikamente zur Behandlung der makularen Degeneration. Daher weist das Primärpackmittel vorzugsweise eine Darreichung zur Behandlung der makularen Degeneration, insbesondere zur Behandlung der altersbedingten makularen Degeneration, der diabetes-induzierten makularen Degeneration, der Wet-AMD, oder einer anderen makularen Degeneration auf.

Besonders bevorzugt ist das Primärpackmittel ausgebildet als Doppelkammerkarpule oder Doppelkammerspritze zur ophthalmischen Anwendung. Alternativ ist es möglich, dass das Primärpackmittel eingerichtet ist als Einkammerkarpule oder Einkammerspritze für die ophthalmische Anwendung.

Das Primärpackmittel wird vorzugsweise in vorsterilisierter Form und/oder in einem Tray oder Tub angeliefert.

Die Aufgabe wird insbesondere auch gelöst, indem ein Verfahren zur Herstellung eines Primärpackmittels geschaffen wird, welches folgende Schritte aufweist: Es wird ein aus einem Kunststoff gebildeter Grundkörper für das Primärpackmittel bereitgestellt, und eine Außenfläche einer Wandung des Grundkörpers wird mit einer gasdichten Beschichtung beschichtet. Besonders bevorzugt wird im Rahmen des Verfahrens ein Primärpackmittel nach einem der zuvor beschriebenen Ausführungsbeispiele hergestellt. Im Rahmen des Verfahrens verwirklichen sich insbesondere die Vorteile, die in Zusammenhang mit dem Primärpackmittel beschrieben wurden. Dabei weist das mittels des Verfahrens hergestellte Primärpackmittel insbesondere nicht die Nachteile auf, welche ein Glas-Primärpackmittel aufweist, wobei es zugleich gasdicht, insbesondere gegenüber zur chemischen Sterilisation verwendeten Stoffen, beispielsweise Ethylenoxid, Sauerstoff, Kohlendioxid, oder Wasserstoffperoxid ist.

Vorzugsweise wird eine gesamte Oberfläche des Grundkörpers, insbesondere eine gesamte Außenfläche desselben, nahtlos mit der gasdichten Beschichtung versehen. Bei einer späteren Terminalsterilisation können dann keine Sterilisationsmittelrückstände über etwaige ungeschützte Bereiche der Außenfläche in einen in dem Primärpackmittel angeordneten Wirk- und/oder Hilfsstoff diffundieren.

Es ist möglich, dass die gasdichte Beschichtung in einem einzigen Beschichtungszyklus hergestellt wird. Es wird aber auch eine Ausführungsform des Verfahrens bevorzugt, bei welchem die gasdichte Beschichtung in einer Mehrzahl von Beschichtungszyklen hergestellt wird, wobei insbesondere eine Mehrzahl gleichartiger oder verschiedener Teilschichten, insbesondere in Hinblick auf ihre chemische Zusammensetzung geringfügig verschiedener Teilschichten, erzeugt werden kann. Dabei ist es insbesondere möglich, die Eigenschaften der gasdichten Beschichtung ausgehend von der Außenfläche flexibel und bedarfsgerecht zu variieren und so eine veränderliche Schicht mit entlang ihrer Höhe - bezogen auf das Primärpackmittel mithin in radialer Richtung - variablen Eigenschaften aufzubauen.

Besonders bevorzugt wird ein Ausführungsbeispiel, bei welchem die gasdichte Beschichtung Silizium oder eine Siliziumverbindung aufweist, wobei ein Sauerstoffgehalt der insbesondere quarz- oder glasartigen Beschichtung entlang einer Höhe der Schicht beziehungsweise einer radialen Erstreckung des Primärpackmittels variiert wird, wobei der Sauerstoffanteil bevorzugt ausgehend von der Außenfläche der Wandung des Grundkörpers nach außen hin zunimmt. Hierdurch wird einerseits eine gute Haftung der gasdichten Beschichtung auf der Außenfläche des aus Kunststoff gebildeten Grundköpers gewährleistet, wobei die quarz- oder glasartigen Eigenschaften der gasdichten Beschichtung nach außen hin zunehmen. Somit wird die gasdichte Beschichtung nach außen hin kratzfester, immobiler und besser geeignet, um eine gute Haftung für Klebe-Etiketten, Farbstoffe oder Markierungen zu vermitteln.

Es wird ein Ausführungsbeispiel des Verfahrens bevorzugt, das sich dadurch auszeichnet, dass der Grundkörper einer Vorbehandlung unterzogen wird. Dabei wird bevorzugt der Kunststoff des Grundkörpers an der Außenfläche funktionalisiert, insbesondere um eine kovalente Bindung der darauf aufbauenden gasdichten Beschichtung zu ermöglichen. Es wird also bevorzugt eine Haftvermittlung zwischen der Außenfläche und der gasdichten Beschichtung realisiert. Bei einer bevorzugten Ausführungsform des Verfahrens wird der Grundkörper durch Plasmabehandlung und/oder durch nasschemische Verfahren vorbehandelt. Dabei entstehen an der Außenfläche funktionale Gruppen, insbesondere radikalische, ionische und/oder stark polare Gruppen, welche eine gute Haftvermittlung zu der gasdichten Beschichtung ermöglichen.

Das beanspruchte Verfahren zeichnet sich dadurch aus, dass eine Innenfläche der Wandung des Grundkörpers mit einer gasdichten Innenbeschichtung beschichtet wird. Diese wird bevorzugt in einem Beschichtungszyklus oder in einer Mehrzahl von Beschichtungszyklen, wie dies bereits in Zusammenhang mit der gasdichten Beschichtung der Außenfläche erläutert wurde, hergestellt. Besonders bevorzugt wird die Innenbeschichtung zumindest bezüglich eines Verfahrensschritts so hergestellt, wie dies zuvor für die Außenbeschichtung erläutert wurde.

Insbesondere ist bevorzugt vorgesehen, dass eine gesamte Oberfläche der Innenfläche nahtlos mit der gasdichten Innenbeschichtung versehen wird. Dies dient - ebenso wie für die Außenfläche erläutert - einer verbesserten Gasdichtheit des Primärpackmittels. Es ist aber auch möglich, die gasdichte Innenbeschichtung lokal oder bereichsweise auszusparen.

Es wird auch eine Ausführungsform des Verfahrens bevorzugt, bei welcher die gasdichte Beschichtung außen und/oder die gasdichte Innenbeschichtung innen zumindest bereichsweise oder vollständig mit wenigstens einer Funktionsschicht, die in Zusammenhang mit der Innenbeschichtung auch als Innenfunktionsschicht bezeichnet wird, versehen wird. Dabei wird im Folgenden der einfacheren Darstellung wegen zum Teil nur allgemein von einer "Funktionsschicht" gesprochen, wobei die Innenfunktionsschicht mitgemeint ist. Die wenigstens eine Funktionsschicht ist vorzugsweise als immobilisierte Schicht ausgebildet. Sie ist bevorzugt eingerichtet, um - als Innenfunktionsschicht - eine Haftreibung von Elastomerstopfen herabzusetzen, um allgemein eine verringerte Empfindlichkeit gegenüber Kratzern oder kosmetischen Defekten zu verleihen, eine verbesserte Haftung von Klebe-Etiketten, Farbstoffen und/oder Markierungen sicherzustellen, und/oder zu unterbinden, dass Inhaltsstoffe von Außenmarkierungen oder Etiketten in den Wirk- und/oder Hilfsstoff im Inneren des Grundkörpers migrieren können. Die Funktionsschicht weist vorzugsweise hydrophobe oder hydrophile Eigenschaften auf. Insbesondere ist es möglich, dass die Funktionsschicht aus wenigstens einem Film gebildet wird, der Polydimethylsiloxan oder eine perfluorierte Verbindung, beispielsweise PTFE, aufweist oder aus einem der genannten Materialien besteht.

Die gasdichte Innenbeschichtung wird bevorzugt, und/ oder die Innenfunktionsschicht erfindungsgemäß bereichsweise, insbesondere lokal, ausgespart. Dies bedeutet, dass in wenigstens einem - vorzugsweise ringförmig umlaufenden - Freibereich der Innenfläche keine gasdichte Innenbeschichtung und/oder keine Innenfunktionsschicht angeordnet wird/werden. Insbesondere bei einem Doppelkammersystem wird als Freibereich bevorzugt ein Bereich, in dem bestimmungsgemäß beim Befüllen und Gefriertrocknen des Primärpackmittels ein Lyophilisat angeordnet wird, insbesondere ein ringförmiger Bereich distal oberhalb eines Mittelstopfenbereichs, in dem bestimmungsgemäß ein Mittelstopfen angeordnet wird, von der gasdichten Beschichtung und/oder der Innenfunktionsschicht ausgespart oder freigehalten.

Besonders bevorzugt wird eine Ausführungsform des Verfahrens, bei welcher die Innenfläche der Wandung des Grundkörpers insgesamt und insbesondere nahtlos mit der gasdichten Innenbeschichtung versehen wird. Entsprechend der vorliegenden Erfindung wird die Innenfunktionsschicht in wenigstens einem Freibereich ausgespart.

Es ist auch möglich, dass die Haftvermittlungsschicht, die gasdichte Beschichtung oder Innenbeschichtung, und/oder die wenigstens eine Funktionsschicht ineinander übergehen, wobei vorzugsweise jede dieser Schichten Silizium aufweist, insbesondere mit einer quarz- oder glasartigen Zusammensetzung, wobei bevorzugt ein Sauerstoffgehalt entlang der Schichtenfolge variiert ist. So wird vorzugsweise die Haftvermittlungsschicht mit einem geringeren Sauerstoffgehalt hergestellt, die gasdichte Beschichtung mit einem mittleren Sauerstoffgehalt, und die wenigstens eine Funktionsschicht mit einem höheren Sauerstoffgehalt.

Die gasdichte Beschichtung und/oder die gasdichte Innenbeschichtung kann/können auch aus dünnen Metall- oder Halbmetallfilmen, beispielsweise aus Gold, Silber, Aluminium, Chrom oder einem anderen geeigneten Material, gebildet werden. Die gasdichte Beschichtung und/oder die gasdichte Innenbeschichtung kann/können auch aus Diamant oder aus Teflon gebildet werden.

Es wird eine Ausführungsform des Verfahrens bevorzugt, die sich dadurch auszeichnet, dass eine Silizium oder eine Siliziumverbindung umfassende Schicht mit - in radialer Richtung gesehen - variablem Sauerstoff-Anteil erzeugt wird. Diese Schicht kann auf der Außenfläche und/oder auf der Innenfläche aufgebaut werden. Dabei variieren die Eigenschaften der Schicht insbesondere von haftvermittelnden Eigenschaften bis hin zu besonders kratz- und defektechten und/oder zum Aufkleben von Etiketten geeigneten Eigenschaften, wie dies zuvor bereits erläutert wurde.

Vorzugsweise wird die gasdichte Beschichtung und/oder die gasdichte Innenbeschichtung hergestellt durch chemische Dampfabscheidung (chemical vapour deposition - CVD), besonders bevorzugt durch plasmaverstärkte chemische Dampfabscheidung (plasma enhanced chemical vapour deposition - PE-CVD). Alternativ oder zusätzlich ist es aber auch möglich, dass die gasdichte Innenbeschichtung und/oder die gasdichte Beschichtung mittels physikalischer Dampfablagerung (physical vapour deposition - PVD) oder durch Sputtern hergestellt wird/werden.

Als Ausgangsmaterial für die gasdichte Beschichtung und/oder die gasdichte Innenbeschichtung wird vorzugsweise ein siliziumhaltiges Material verwendet, besonders bevorzugt Hexamethyldisiloxan (HMDSO). Dieses wird vorzugsweise mittels einer plasmaverstärkten chemischen Dampfablagerung (plasma enhanced chemical vapour deposition - PE-CVD) aufgebracht. Dabei wird vorzugsweise dem Hexamethyldisiloxan Sauerstoff beigemischt, wobei ein Sauerstoffanteil im Laufe der Beschichtung erhöht wird, sodass graduell eine zunehmend glas- oder quarzartige Beschichtung gebildet wird. Dabei kann der Sauerstoff-Anteil in Form eines kontinuierlichen Gradienten oder auch stufenförmig erhöht werden.

Es ist auch möglich, dass als Ausgangsmaterial für die gasdichte Beschichtung und/oder die gasdichte Innenbeschichtung ein Metall oder Halbmetall verwendet wird, beispielsweise Gold, Silber, Aluminium, Chrom, oder ein anderes geeignetes Material. Dabei können insbesondere - abhängig von der Schichtdicke - semitransparente Schichten und/oder spiegelnde Schichten erzeugt werden.

Schließlich wird eine Ausführungsform des Verfahrens bevorzugt, bei welcher wenigstens eine Schicht ausgewählt aus der gasdichten Beschichtung und der gasdichten Innenbeschichtung quervernetzt wird. Hierbei werden - insbesondere durch eine Plasmabehandlung - funktionelle Gruppen, reaktive Gruppen, radikalische Gruppen, ionische Gruppen, oder Doppelbindungen bereitgestellt, die miteinander reagieren und so ein dreidimensionales Netzwerk innerhalb der Schicht bilden können. Es ist möglich, dass die Vernetzung mittels UV-Bestrahlung aktiviert wird. Hierdurch kann die Molmasse der Schicht erhöht werden, und die Schicht kann sehr effektiv immobilisiert werden.

Vorzugsweise werden die gasdichte Innenbeschichtung und die gasdichte Beschichtung an der Außenfläche in einem einzigen Verfahrensschritt aufgebracht.

Die Beschreibung des Primärpackmittels einerseits und des Verfahrens andererseits sind komplementär zueinander zu verstehen. Merkmale des Primärpackmittels, die explizit oder implizit in Zusammenhang mit dem Verfahren erläutert wurden, sind bevorzugt einzeln oder miteinander kombiniert Merkmale eines bevorzugten Ausführungsbeispiels des Primärpackmittels. Verfahrensschritte, die explizit oder implizit in Zusammenhang mit dem Primärpackmittel erläutert wurden, sind bevorzugt einzeln oder miteinander kombiniert Schritte einer bevorzugten Ausführungsform des Verfahrens. Dieses zeichnet sich bevorzugt durch wenigstens einen Verfahrensschritt aus, der durch wenigstens ein Merkmal des Primärpackmittels bedingt ist. Das Primärpackmittel zeichnet sich bevorzugt durch wenigstens ein Merkmal aus, welches durch wenigstens einen Schritt einer bevorzugten Ausführungsform des Verfahrens bedingt ist.

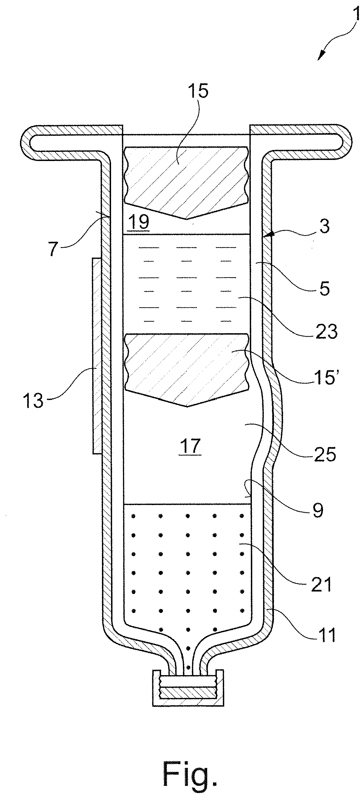

Die Erfindung wird im Folgenden anhand der Zeichnung näher erläutert. Dabei zeigt die einzige Figur eine schematische Darstellung eines Ausführungsbeispiels eines Primärpackmittels.

Die einzige Figur zeigt eine schematische Darstellung eines Ausführungsbeispiels eine Primärpackmittels 1 für medizinische Zubereitungen, welches einen Grundkörper 3 aufweist, der aus einem Kunststoff gebildet ist, wobei der Grundkörper 3 vorzugsweise aus einem Kunststoff besteht. Er weist eine Wandung 5 auf, die eine Außenfläche 7 und eine Innenfläche 9 aufweist. Bei derartigen Primärpackmitteln 1 aus Kunststoff besteht generell das Problem, dass bei einer chemischen Sterilisation zur Gewährleistung einer Außensterilität verwendete Reagenzien durch den Kunststoff des Grundkörpers 3 in das Innere des Primärpackmittels 1 eindringen und dort vorhandene Wirk- und/oder Hilfsstoffe kontaminieren können.

Um dies zu vermeiden, ist die Außenfläche 7 hier mit einer gasdichten Beschichtung 11 versehen. Dabei ist insbesondere vorgesehen, dass die gesamte Außenfläche 7 nahtlos mit der gasdichten Beschichtung 11 versehen ist, damit bei einer Terminalsterilisation des vorgefüllten Primärpackmittels 1 keine Sterilisationsmittelrückstände über etwaige ungeschützte Bereiche des Grundkörpers 3 in in dem Primärpackmittel 1 vorhandene Wirk- und/oder Hilfsstoffe diffundieren können.

Die gasdichte Beschichtung wird vorzugsweise mittels chemischer Dampfauftragung (chemical vapour deposition - CVD), insbesondere plasmaverstärkter chemischer Dampfauftragung (plasma enhanced chemical vapour deposition - PE-CVD), durch physikalische Dampfauftragung (physical vapour deposition - PVD), oder durch Sputtern aufgebracht.

Vorzugsweise wird vor dem Aufbringen der gasdichten Beschichtung 11 auf der Außenfläche 7 eine in der Figur nicht dargestellte Haftvermittlerschicht aufgebracht.

Die gasdichte Beschichtung 11 weist vorzugsweise ein Metall oder ein Halbmetall auf, wobei sie besonders bevorzugt aus wenigstens einem Metall- oder Halbmetallfilm besteht. Als metallische oder halbmetallische Materialien kommen insbesondere Gold, Silber, Aluminium, Chrom, oder andere geeignete Materialien infrage. Alternativ oder zusätzlich weist die gasdichte Beschichtung 11 vorzugsweise Silizium oder eine Siliziumverbindung - insbesondere mit glas- oder quarzartiger Zusammensetzung - auf oder besteht aus Silizium oder einer Siliziumverbindung - insbesondere mit quarz- oder glasartiger Zusammensetzung. Es ist auch möglich, dass die gasdichte Beschichtung 11 Diamant aufweist oder aus Diamant besteht. Alternativ oder zusätzlich ist es möglich, dass die gasdichte Beschichtung 11 Teflon aufweist oder aus Teflon besteht.

Die gasdichte Beschichtung und die Haftvermittlerschicht können auch in einem einzigen Beschichtungsvorgang und aus einem gleichen Ausgangsmaterial hergestellt werden, bevorzugt indem eine chemische Zusammensetzung der gasdichten Beschichtung 11 während des Beschichtungsprozesses variiert wird.

Die gasdichte Beschichtung 11 wird vorzugsweise aus Hexamethyldisiloxan (HMDSO) gebildet, wobei sie insbesondere durch Plasmabeschichtung mit Argon als Trägergas unter Beimischung von Sauerstoff aufgebracht wird. Dabei kann insbesondere ein Sauerstoffanteil während des Beschichtungsprozesses variiert werden, wobei der Sauerstoffanteil insbesondere - kontinuierlich oder stufenweise - erhöht werden kann. So ist es möglich, zunächst auf die Außenfläche 7 - quasi als Haftvermittlerschicht - eine Schicht aufzubringen, welche Silizium mit einem geringeren Sauerstoffanteil aufweist, wobei der Sauerstoffanteil dann nach außen hin erhöht wird, sodass die gasdichte Beschichtung 11 nach außen hin zunehmend quarz- oder glasartige Eigenschaften aufweist.

Der Grundkörper 3 weist vorzugsweise ein zyklisches Olefin-Polymer (COP), ein zyklisches Olefin-Copolymer (COC) oder Polycarbonat (PC) auf, oder besteht aus einem der genannten Materialien.

In der Figur ist auch dargestellt, dass auf der gasdichten Beschichtung 11 bereichsweise eine Funktionsschicht 13 angeordnet ist. Diese ist insbesondere eingerichtet, um möglichst günstige Hafteigenschaften für Klebe-Etiketten bereitzustellen. Alternativ oder zusätzlich ist es möglich, dass die Funktionsschicht 13 eine geringere Empfindlichkeit gegenüber Kratzern oder kosmetischen Defekten aufweist, als die gasdichte Beschichtung 11. Dabei kann auch die Funktionsschicht 13 integral mit der gasdichten Beschichtung 11 ausgebildet werden, insbesondere indem der Sauerstoff-Anteil einer Beschichtung, die Silizium aufweist, noch weiter erhöht wird. Somit ist die Funktionsschicht 13 besonders bevorzugt eine quarz- oder glasartige Schicht, die einen höheren Sauerstoffanteil aufweist, als die gasdichte Beschichtung 11.

Die gasdichte Beschichtung 11 und/oder die Funktionsschicht 13 sind bevorzugt immobilisiert, wobei insbesondere eine Quervernetzung innerhalb wenigstens einer dieser Schichten vorgesehen sein kann.

Der Grundkörper 3 weist vorzugsweise auch auf der Innenfläche 9 eine in der Figur nicht dargestellte gasdichte Innenbeschichtung auf.

Dabei ist es auch hier möglich, dass zwischen der Innenfläche 9 und der gasdichten Innenbeschichtung eine Haftvermittlerschicht angeordnet ist. Weiterhin ist es möglich, dass auf der gasdichten Innenbeschichtung zumindest bereichsweise eine Innenfunktionsschicht angeordnet ist. Diese ist dann insbesondere eingerichtet, um eine Haftreibung von Elastomerstopfen 15, 15' herabzusetzen und somit insbesondere eine vereinfachte Dosierung auch kleiner Volumina zu ermöglichen. Weiterhin ist die gasdichte Innenbeschichtung bevorzugt immobilisiert, sodass sie insbesondere keine messbaren Partikel oder Fremdstoffe - wie beispielsweise Silikontropfen - an in dem Primärpackmittel 1 angeordnete Wirk- und/oder Hilfsstoffe abgibt. Weiterhin weist die gasdichte Innenbeschichtung und/oder die wenigstens eine Innenfunktionsschicht auf der gasdichten Innenbeschichtung vorzugsweise hydrophobe oder hydrophile Eigenschaften auf. Dabei dienen diese Eigenschaften insbesondere einer vollständigen Entleerung des Primärpackmittels 1, wobei sie bevorzugt auf in dem Primärpackmittel 1 angeordnete Wirk- und/oder Hilfsstoffe abgestimmt sind. Eine hydrophobe Schicht ist vorzugsweise aus wenigstens einem Film aufgebaut, welcher auf Polydimethylsiloxan oder einer perfluorierten Verbindung basiert, beispielsweise auf Polytetrafluorethylen (PTFE). Eine hydrophile Schicht kann beispielsweise durch plasmainduzierte Polymerisation von Acrylsäure hergestellt werden. Dabei entstehen an der Oberfläche der Schicht Carboxylgruppen.

Weiterhin schützt die gasdichte Innenbeschichtung und/oder eine auf diese aufgebrachte Innenfunktionsschicht das Primärpackmittel 1 vorzugsweise vor einer Verwitterung bei einem Gefriertrocknungsprozess, sodass hierbei keine Partikel in in dem Primärpackmittel 1 angeordnete Wirk- und/oder Hilfsstoffe gelangen können.

Die gasdichte Innenbeschichtung und/oder die Innenfunktionsschicht kann/können auch in einem Freibereich ausgespart sein, insbesondere um eine Interaktion mit einem Gefriertrockengut oder Lyophilisat zu vermeiden. Insbesondere kann nur die Innenfunktionsschicht in dem Freibereich ausgespart werden.

Bevorzugt werden die innenliegenden Schichten und die an der Außenfläche 7 aufgebauten Schichten gemeinsam in einem einzigen Verfahrensschritt aufgebracht.

Das in der Figur dargestellte Ausführungsbeispiel des Primärpackmittels 1 ist als Doppelkammersystem ausgebildet. Dabei weist es eine erste, distale Kammer 17 und eine zweite, proximale Kammer 19 auf. In der ersten, distalen Kammer 17 ist ein erster Wirk- und/oder Hilfsstoff 21 angeordnet, insbesondere ein lyophilisierter oder pulverförmiger Wirk- und/oder Hilfsstoff. In der zweiten, proximalen Kammer 19 ist ein zweiter Wirk- und/oder Hilfsstoff 23 angeordnet, insbesondere ein flüssiges Lösungsmittel. Die Trennung des ersten Wirk- und/oder Hilfsstoffs 21 von dem zweiten Wirk- und/oder Hilfsstoff 23 dient insbesondere einer verlängerten Lagerfähigkeit der medizinischen Zubereitung.

In für sich genommen bekannter Weise sind die erste, distale Kammer 17 und die zweite, proximale Kammer 19 durch einen Bypass 25 miteinander verbunden. Ein erster Elastomerstopfen 15 dient als Endstopfen der Durchführung einer Injektion, wobei ein zweiter Elastomerstopfen 15' als Mittelstopfen in einem Lagerzustand des Primärpackmittels 1 derart im Bereich des Bypasses 25 angeordnet ist, dass dieser versperrt ist, wodurch die erste Kammer 17 von der zweiten Kammer 19 getrennt ist. Um das Primärpackmittel 1 zu konditionieren, kann der erste Elastomerstopfen 15 in axialer Richtung - in der Figur nach unten - verlagert werden, wodurch der Druck in der zweiten, proximalen Kammer 19 erhöht wird, so dass sich auch der zweite Elastomerstopfen 15' in distaler Richtung bewegt. Dabei wird schließlich der Bypass 25 freigegeben, sodass der zweite Wirk- und/oder Hilfsstoff 23, insbesondere das Lösungsmittel, von der zweiten Kammer 19 in die erste Kammer 17 überströmen kann, wo er sich mit dem ersten Wirk- und/oder Hilfsstoff 21 vermischt und/oder diesen auflöst. Durch eine weitere, distale Verlagerung des ersten Elastomerstopfens 15 kann dann eine Injektion erfolgen.

Alternativ ist es möglich, dass das Primärpackmittel 1 als Einkammerkarpule, als Einkammerspritze, oder als Doppelkammerspritze ausgebildet ist. Besonders bevorzugt ist das Primärpackmittel 1 eingerichtet für ophthalmische Anwendungen, wobei es insbesondere Wirk- und/oder Hilfsstoffe 21, 23 aufweist, die eingerichtet sind zur Behandlung von Augenkrankheiten, insbesondere der makularen Degeneration, ganz besonders der altersbedingten, diabetes-induzierten, oder nassen makularen Degeneration. Dabei ist das Primärpackmittel 1 insbesondere eingerichtet, um eine Injektion direkt in den Glaskörper des menschlichen Auges durchzuführen, wozu eine Außensterilität des Primärpackmittels 1 zwingend gewährleistet sein muss.

Weiterhin ist das Primärpackmittel 1 bevorzugt eingerichtet zur Verabreichung geringer Füllvolumina von vorzugsweise weniger als oder höchstens 200 µl, wobei insbesondere nicht mehr als 50 µl in den Glaskörper des menschlichen Auges injiziert werden sollen. Es ist möglich, dass das Primärpackmittel 1 ein Nennvolumen von 1 ml oder 0,5 ml aufweist.

Insgesamt zeigt sich, dass mit dem Primärpackmittel 1 ein Primärpackmittel bereitgestellt wird, welches nicht die in Zusammenhang mit Glas-Primärpackmitteln bestehenden Nachteile einer Gefahr von sich ablösenden, mikroskopischen Glasteilchen sowie einer nicht tolerierbaren Anzahl subvisueller Partikel in einem in dem Primärpackmittel 1 vorhandenen Wirk- und/oder Hilfsstoff aufweist, wobei es zugleich mittels chemischer Sterilisation in befülltem Zustand terminal sterilisierbar ist.

## Patentansprüche

1. Primärpackmittel (1) für medizinische Zubereitungen, mit
- einem Grundkörper (3), der einen Kunststoff aufweist, wobei
- der Grundkörper (3) wenigstens eine Wandung (5) mit einer Außenfläche (7) und wenigstens einer Innenfläche (9) aufweist, wobei
die Außenfläche (7) mit einer gasdichten Beschichtung (11) versehen ist, wobei die Innenfläche (9) mit einer gasdichten Innenbeschichtung versehen ist, und wobei auf der gasdichten Innenbeschichtung bereichsweise eine Innenfunktionsschicht angeordnet ist, **dadurch gekennzeichnet, dass** die Innenfunktionsschicht in wenigstens einem Freibereich ausgespart ist.

2. Primärpackmittel (1) nach Anspruch 1, **dadurch gekennzeichnet, dass** die gasdichte Beschichtung (11) eine Schicht aufweist, die ein Metall oder ein Halbmetall aufweist, oder aus einem Metall oder Halbmetall besteht, oder die Silizium - vorzugsweise mit glas- oder quarzartiger Zusammensetzung - aufweist, oder aus einer Siliziumverbindung - vorzugsweise mit glas- oder quarzartiger Zusammensetzung - besteht.

3. Primärpackmittel (1) nach einem der vorhergehenden Ansprüche, **dadurch gekennzeichnet, dass** der Grundkörper (3) einen durchsichtigen Kunststoff aufweist oder aus einem durchsichtigen Kunststoff besteht, wobei der durchsichtige Kunststoff vorzugsweise ausgewählt ist aus einer Gruppe bestehend aus einem zyklischen Olefin-Polymer, einem zyklischen Olefin-Copolymer und Polycarbonat.

4. Primärpackmittel (1) nach einem der vorhergehenden Ansprüche, **dadurch gekennzeichnet, dass** zwischen der Außenfläche (7) und der gasdichten Beschichtung (11) eine Haftvermittlungsschicht angeordnet ist.

5. Primärpackmittel (1) nach einem der vorhergehenden Ansprüche, **dadurch gekennzeichnet, dass** auf der gasdichten Beschichtung (11) zumindest bereichsweise eine Funktionsschicht (13) angeordnet ist, wobei die zumindest eine Funktionsschicht (13) als immobilisierte Schicht ausgebildet und/oder eingerichtet ist, um eine verringerte Empfindlichkeit gegenüber Kratzern oder kosmetischen Defekten zu verleihen, und/oder um eine verbesserte Haftung von Klebe-Etiketten, Farbstoffen und/oder Markierungen sicherzustellen, und/oder um zu unterbinden, dass Inhaltsstoffe von Außenmarkierungen oder Etiketten in einen Wirk- und/oder Hilfsstoff in einem Inneren des Grundkörpers (3) migrieren können.

6. Primärpackmittel (1) nach einem der vorhergehenden Ansprüche, **dadurch gekennzeichnet, dass** die Innenfunktionsschicht hydrophobe oder hydrophile Eigenschaften aufweist.

7. Primärpackmittel (1) nach einem der vorhergehenden Ansprüche, **dadurch gekennzeichnet, dass** das Primärpackmittel (1) als Doppelkammerkarpule, als Doppelkammerspritze, als Einkammerkarpule oder als Einkammerspritze ausgebildet ist.

8. Primärpackmittel (1) nach einem der vorhergehenden Ansprüche, **dadurch gekennzeichnet, dass** das Primärpackmittel (1) eingerichtet ist für eine ophthalmische Anwendung, wobei ein Füllvolumen des Primärpackmittels (1) 1 ml, oder 0,5 ml, oder höchstens 200 µl beträgt.

9. Verfahren zur Herstellung eines Primärpackmittels (1), insbesondere zur Herstellung eines Primärpackmittels nach einem der Ansprüche 1 bis 8, mit folgenden Schritten:
- Bereitstellen eines einen Kunststoff aufweisenden Grundkörpers (3);
- Beschichten einer Außenfläche (7) einer Wandung (5) des Grundkörpers (3) mit einer gasdichten Beschichtung (11);
- Beschichten einer Innenfläche der Wandung (5) des Grundkörpers (3) mit einer gasdichten Innenbeschichtung (9), und
- Anordnen einer Innenfunktionsschicht bereichsweise auf der gasdichten Innenbeschichtung (9), wobei die Innenfunktionsschicht zumindest in einem Freibereich ausgespart wird.

10. Verfahren nach Anspruch 9, **dadurch gekennzeichnet, dass** die Außenfläche (7) und/oder eine Innenfläche (9) der Wandung (5) einer Vorbehandlung, insbesondere zur Haftvermittlung für die gasdichte Beschichtung (11) oder die gasdichte Innenbeschichtung, unterzogen wird.

11. Verfahren nach einem der Ansprüche 9 und 10, **dadurch gekennzeichnet, dass** auf der gasdichten Beschichtung (11) zumindest bereichsweise eine Funktionsschicht (13) angeordnet wird, wobei die Funktionsschicht (13) als immobilisierte Schicht ausgebildet und/oder eingerichtet ist, um eine verringerte Empfindlichkeit gegenüber Kratzern oder kosmetischen Defekten zu verleihen, und/oder um eine verbesserte Haftung von Klebe-Etiketten, Farbstoffen und/oder Markierungen sicherzustellen, und/oder um zu unterbinden, dass Inhaltsstoffe von Außenmarkierungen oder Etiketten in einen Wirk- und/oder Hilfsstoff in einem Inneren des Grundkörpers (3) migrieren können.

12. Verfahren nach einem der Ansprüche 9 bis 11, **dadurch gekennzeichnet, dass** als gasdichte Beschichtung (11) oder als gasdichte Innenbeschichtung eine Silizium aufweisende Schicht mit entlang ihrer Höhe variierendem Sauerstoff-Anteil erzeugt wird.

13. Verfahren nach einem der Ansprüche 9 bis 12, **dadurch gekennzeichnet, dass** wenigstens eine Schicht, ausgewählt aus der gasdichten Beschichtung (11), der gasdichten Innenbeschichtung, einer Funktionsschicht (13) oder einer Innenfunktionsschicht, einer Quervernetzung unterzogen wird.

## Claims

1. A primary packaging (1) for medical preparations, having
- a base body (3) comprising plastic material, wherein
- the base body (3) has at least one wall (5) including an outer surface (7) and at least one inner surface (9), wherein
the outer surface (7) is provided with a gas tight coating (11), wherein the inner surface (9) is provided with a gas tight inner coating, and wherein an inner functional layer is arranged in sections on the gas tight inner coating, **characterized in that** the inner functional layer is omitted in at least one free region.

2. The primary packaging (1) according to claim 1, **characterized in that** the gas tight coating (11) has a layer, which comprises a metal or a metalloid, or is made up of a metal or metalloid, or which comprises silicon - preferably having a vitreous or quartz-like composition - or is made up of a silicon compound - preferably having a vitreous or quartz-like composition.

3. The primary packaging (1) according to one of the preceding claims, **characterized in that** the base body (3) comprises a transparent plastic material or is made up of a transparent plastic material, wherein the transparent plastic material preferably is selected from a group consisting of a cyclic olefin polymer, a cyclic olefin copolymer and polycarbonate.

4. The primary packaging (1) according to one of the preceding claims, **characterized in that** an adhesion-promoting layer is arranged between the outer surface (7) and the gas tight coating (11).

5. The primary packaging (1) according to one of the preceding claims, **characterized in that** a functional layer (13) is arranged at least in sections on the gas tight coating (11), wherein the at least one functional layer (13) is formed and/or configured as an immobilized layer to provide a reduced susceptibility to scratches or cosmetic defects, and/or to ensure an improved adhesion of adhesive labels, dyes and/or markings, and/or to inhibit that constituents of outer markings or labels can migrate into an active substance and/or adjuvant in an interior of the base body (3).

6. The primary packaging (1) according to one of the preceding claims, **characterized in that** the inner functional layer comprises hydrophobic or hydrophilic properties.

7. The primary packaging (1) according to one of the preceding claims, **characterized in that** the primary packaging (1) is configured as a dual chamber cartridge, as a dual chamber syringe, as a single chamber cartridge or as a single chamber syringe.

8. The primary packaging (1) according to one of the preceding claims, **characterized in that** the primary packaging (1) is configured for an ophthalmic application, wherein a filling volume of the primary packaging (1) is 1 ml, or 0.5 ml or at most 200 µl.

9. A method for manufacturing a primary packaging (1), in particular for manufacturing a primary packaging according to one of claims 1 through 8, comprising the following steps:
- providing a base body (3) comprising a plastic material;
- coating an outer surface (7) of a wall (5) of the base body (3) with a gas tight coating (11);
- coating an inner surface of the wall (5) of the base body (3) with a gas tight inner coating (9), and
- arranging an inner functional layer in sections on the gas tight inner coating (9), wherein the inner functional layer is omitted in at least one free region.

10. The method according to claim 9, **characterized in that** the outer surface (7) and/or an inner surface (9) of the wall (5) is/are pretreated, in particular to promote adhesion for the gas tight coating (11) or the gas tight inner coating.

11. The method according to one of claims 9 and 10, **characterized in that** a functional layer (13) is arranged at least in sections on the gas tight coating (11), wherein the functional layer (13) is formed and/or configured as an immobilized layer to provide a reduced susceptibility to scratches or cosmetic defects, and/or to ensure an improved adhesion of adhesive labels, dyes and/or markings, and/or to inhibit that constituents of outer markings or labels can migrate into an active substance and/or adjuvant in an interior of the base body (3).

12. The method according to one of claims 9 through 11, **characterized in that** a layer comprising silicon and having a varied percentage of oxygen along its height is generated as the gas tight coating (11) or as the gas tight inner coating.

13. The method according to one of claims 9 through 12, **characterized in that** at least one layer, selected from the gas tight coating (11), the gas tight inner coating, a functional layer (13) or an inner functional layer, is crosslinked.

## Revendications

1. Emballage primaire (1) pour préparations médicales, avec
- un corps de base (3) qui présente un plastique, dans lequel
- le corps de base (3) présente au moins une paroi (5) avec une face externe (7) et au moins une face interne (9), dans lequel
la face externe (7) est pourvue d'un revêtement étanche au gaz (11), dans lequel la face interne (9) est pourvue d'un revêtement interne étanche au gaz, et dans lequel une couche fonctionnelle interne est disposée par région sur le revêtement interne étanche au gaz, **caractérisé en ce que** la couche fonctionnelle interne est évidée dans au moins une région libre.

2. Emballage primaire (1) selon la revendication 1, **caractérisé en ce que** le revêtement étanche au gaz (11) présente une couche qui présente un métal ou un semi-métal, ou se compose d'un métal ou semi-métal, ou qui présente du silicium, de préférence avec une composition de type verre ou quartz, ou se compose d'un composé de silicium, de préférence avec une composition de type verre ou quartz.

3. Emballage primaire (1) selon une des revendications précédentes, **caractérisé en ce que** le corps de base (3) présente un plastique transparent ou se compose d'un plastique transparent, dans lequel le plastique transparent est de préférence sélectionné parmi un groupe constitué d'un polymère d'oléfine cyclique, d'un copolymère d'oléfine cyclique et de polycarbonate.

4. Emballage primaire (1) selon une des revendications précédentes, **caractérisé en ce qu'**une couche promotrice d'adhésion est disposée entre la face externe (7) et le revêtement étanche au gaz (11).

5. Emballage primaire (1) selon une des revendications précédentes, **caractérisé en ce qu'**une couche fonctionnelle (13) est disposée au moins par région sur le revêtement étanche au gaz (11), dans lequel l'au moins une couche fonctionnelle (13) est réalisée et/ou agencée en tant que couche immobilisée pour donner une sensibilité réduite aux rayures ou défauts cosmétiques, et/ou pour garantir une adhésion améliorée d'étiquettes adhésives, colorants et/ou marquages, et/ou pour empêcher que des ingrédients de marquages externes ou étiquettes ne puissent migrer dans une substance active et/ou un adjuvant dans un intérieur du corps de base (3).

6. Emballage primaire (1) selon une des revendications précédentes, **caractérisé en ce que** la couche fonctionnelle interne présente des propriétés hydrophobes ou hydrophiles.

7. Emballage primaire (1) selon une des revendications précédentes, **caractérisé en ce que** l'emballage primaire (1) est réalisé en tant que carpule à double chambre, en tant que seringue à double chambre, en tant que carpule à une chambre ou en tant que seringue à une chambre.

8. Emballage primaire (1) selon une des revendications précédentes, **caractérisé en ce que** l'emballage primaire (1) est agencé pour une application ophtalmique, dans lequel un volume de remplissage de l'emballage primaire (1) est de 1 ml ou 0,5 ml ou 200 µl au maximum.

9. Procédé de fabrication d'un emballage primaire (1), notamment de fabrication d'un emballage primaire selon une des revendications 1 à 8, avec les étapes suivantes :
- mise à disposition d'un corps de base (3) présentant un plastique ;
- enrobage d'une face externe (7) d'une paroi (5) du corps de base (3) avec un revêtement étanche au gaz (11) ;
- enrobage d'une face interne de la paroi (5) du corps de base (3) avec un revêtement interne étanche au gaz (9), et
- disposition d'une couche fonctionnelle interne par région sur le revêtement interne étanche au gaz (9), dans lequel la couche fonctionnelle interne est évidée au moins dans une région libre.

10. Procédé selon la revendication 9, **caractérisé en ce que** la face externe (7) et/ou une face interne (9) de la paroi (5) est soumise à un prétraitement, notamment pour la promotion d'adhésion pour le revêtement étanche au gaz (11) ou le revêtement interne étanche au gaz.

11. Procédé selon une des revendications 9 et 10, **caractérisé en ce qu'**une couche fonctionnelle (13) est disposée au moins par région sur le revêtement étanche au gaz (11), dans lequel la couche fonctionnelle (13) est réalisée et/ou agencée en tant que couche immobilisée pour donner une sensibilité réduite aux rayures ou défauts cosmétiques, et/ou pour garantir une adhésion améliorée d'étiquettes adhésives, colorants et/ou marquages, et/ou pour empêcher que des ingrédients de marquages externes ou étiquettes ne puissent migrer dans une substance active et/ou un adjuvant dans un intérieur du corps de base (3).

12. Procédé selon une des revendications 9 à 11, **caractérisé en ce qu'**une couche présentant du silicium avec une proportion d'oxygène variant le long de sa hauteur est générée en tant que revêtement étanche au gaz (11) ou en tant que revêtement interne étanche au gaz.

13. Procédé selon une des revendications 9 à 12, **caractérisé en ce qu'**au moins une couche sélectionnée parmi le revêtement étanche au gaz (11), le revêtement interne étanche au gaz, une couche fonctionnelle (13) ou une couche fonctionnelle interne est soumise à une réticulation transversale.
